# EUROPEAN PATENT APPLICATION

(11) **EP 2 564 814 A1**
(43) Date of publication of application: **06.03.2013**
(21) Application number: 12250146.3
(22) Date of filing: 31.08.2012
(51) Int. Cl.: A61F 2/40

(54) **Stemless shoulder implant assembly**

(30) Priority: 31.08.2011 GB 201115054
(71) Applicant: Rangan Implants and Procedures Ltd., Middlesbrough TS4 2NS (GB)
(72) Inventor: Rangan, Amar, Middlesbrough TS4 2NS (GB)
(74) Representative: Hutchinson, Claire Louise

(57) **Abstract**

A modular implant assembly is provided for a shoulder joint having a humerus and a glenoid. The assembly comprises a base plate and a joint head having a convex outer surface and a generally planar base. The joint head is configured to couple with said base plate. The base plate is configured with means for fixation to the humerus and further comprises at least three radially displaced screws configured to attach said base plate to the humerus, wherein said screws attach to the peripheral cortical region of the humerus.

## Description

### CROSS REFERENCE TO RELATED APPLICATIONS

This application claims priority from United Kingdom patent application No 11 15 054.7, filed 31 August 2011, the whole contents of which are included herein by way of reference.

### BACKGROUND OF THE INVENTION

### 1. Field of the Invention

The present invention relates to a modular implant assembly for replacement of a shoulder joint having a humerus and a glenoid, the implant assembly being attached to the humerus by stemless means. The modular implant assembly of the present invention may be articulated with the patient's own glenoid or a prosthetic glenoid. The present invention also relates to an assembly of implant components for a glenohumeral shoulder joint.

### 2. Description of the Related Art

Traditional methods of implanting anatomical prosthetic shoulder assemblies involve anchoring a metal dome-shaped cap to the patient's humerus by a stem that is inserted into the central canal of their humerus. There are many disadvantages to these traditional methods. In particular, a large amount of bone removed needs to be removed from the central region of the humerus for the insertion of the stem. This weakens the humeral bone and the stem can then cause the bone to fracture at implant. Furthermore, the bone at the central region of the humerus is soft and therefore not ideal for preventing the implant from sinking.

Alternative anatomical prosthetic shoulder assemblies that do not require a stem are known, and in these, the existing ball of the joint is capped. A cap is placed over the end of the patient's humeral head and held in place with a central peg. However, a drawback here is that the ball of the joint is not taken away and this can make subsequent access to the joint socket difficult when it is necessary to intervene, creating problems in terms of replacing the socket in a reliable and reproducible way.

Unlike the prior art, the present invention provides an implant assembly for fixation to the humerus without a requirement for either a stem or cement. The present invention provides a less invasive implant assembly and therefore does not have the disadvantages of the prior art. The present invention relates to a modular means of replacing the ball of the shoulder joint and anchoring it to the joint. The implant assembly as described by the present invention may be articulated with the patient's own glenoid or a replacement glenoid.

### BRIEF SUMMARY OF THE INVENTION

According to an aspect of the present invention, there is provided a modular implant assembly for a shoulder joint having a humerus and a glenoid, the assembly comprising a base plate, a joint head having a convex outer surface and a generally planar base and which is configured to couple with said base plate, and wherein said base plate is configured with stemless means for fixation to the humerus. The base plate further comprises at least three radially displaced screws configured to attach said base plate to the humerus, wherein said screws attach to the peripheral cortical regions of the humerus.

### BRIEF DESCRIPTION OF THE SEVERAL VIEWS OF THE DRAWINGS

Figure 1 shows a person suffering from arthritis having had a stemmed humeral implant fitted in her right shoulder.
Figure 2 shows a cross sectional view of a shoulder joint having a humerus and a glenoid in a healthy person.
Figures 3a and 3b show the prior art. A traditional anatomical glenohumeral shoulder replacement is shown in Figure 3a, in which the humeral component is attached to the humerus by a stem. Figure 3b shows an individual humeral stem.
Figures 4a and 4b each show an implant assembly for a shoulder joint embodying the present invention. Figure 4a shows the joint head fixed to the humerus. Figure 4b shows the joint head fixed to the humerus, in association with a modular glenoid component system.
Figure 5 shows a side view of the implant assembly embodying the present invention;
Figure 6a shows a side view of the implant assembly embodying the present invention.
Figure 6b shows a top view of one side of the implant assembly embodying the present invention.
Figure 6c shows screws as means of attachment of the implant assembly to the humerus.
Figure 7 shows a perspective view of the joint head bounded by a convex outer surface and a generally planar base;
Figure 8 shows a cross sectional view of an implant assembly embodying the present invention;
Figure 9 shows a cross sectional view of an implant assembly embodying the present invention;
Figure 10a shows a perspective view of the implant assembly embodying the present invention;
Figure 10b shows a different perspective view of the implant assembly embodying the present invention.

### DETAILED DESCRIPTION OF THE PREFERRED EMBODIMENTS

### Figure 1

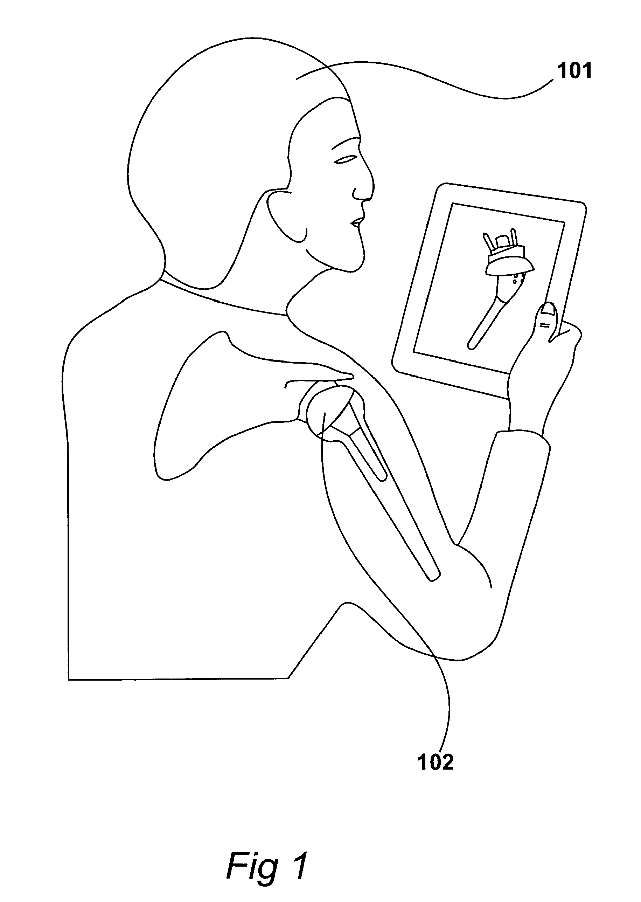

The shoulder is a ball and socket joint, the ball being the head of the humerus and the socket being the glenoid. A normal shoulder joint having a humerus and a glenoid may degenerate over time as a result of arthritis or other age-related conditions. When shoulder joint degeneration advances such that the shoulder no longer functions or movement is extremely painful, the normal shoulder joint may be replaced with a prosthetic shoulder joint. Figure 1 shows person 101 suffering from arthritis with a prosthetic shoulder joint 102 implanted in her right shoulder. Shoulder replacement has provided good clinical results over the past 15 years and improved quality of life of patients suffering from shoulder pain.

### Figure 2

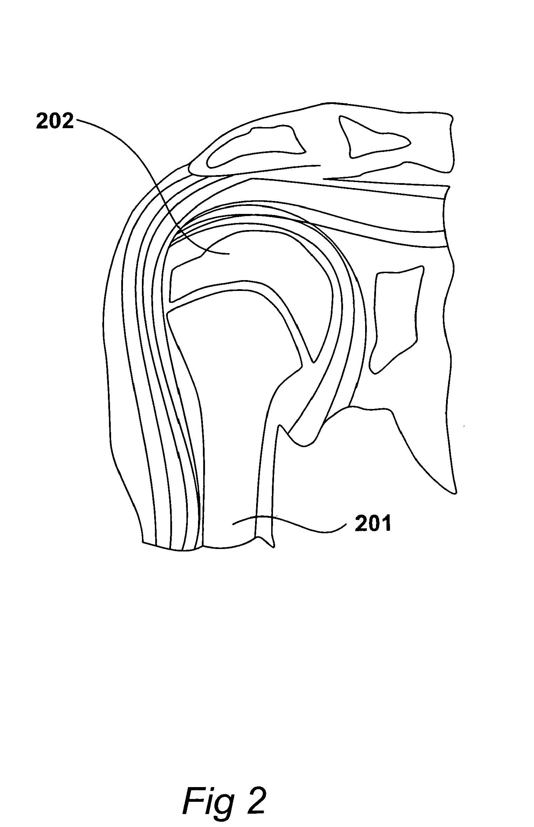

A cross-sectional view of a normal shoulder joint having a humerus 201 and a glenoid 202 in a healthy person is shown in Figure 2. The shoulder is a ball and socket joint, the ball being the head of the humerus and the socket being the glenoid.

### Figure 3

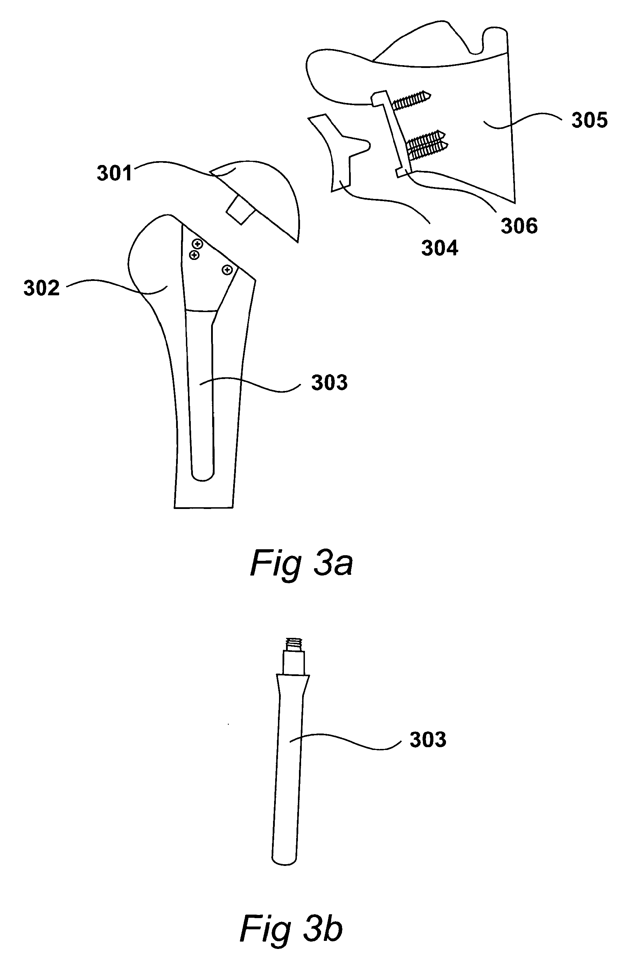

The prior art is shown in Figures 3a and 3b.

A traditional anatomical glenohumeral shoulder replacement is shown in Figure 3a, in which the prosthetic humeral component 301 is attached to the humerus 302 by a stem 303. During this shoulder replacement the patient's humeral head is resected or cut, creating a cavity in the central intramedullary canal of the patient's humerus. The prosthetic humeral component 301 is then attached to the patient's humerus 302 by means of stem 303 inserted into this central canal of their humerus 302. The prosthetic humeral component 301 is also known as prosthetic humeral head 301.

Prosthetic humeral component 301 may articulate with the patient's own glenoid if it has not degenerated or a prosthetic glenoid. When articulating with a prosthetic glenoid, glenoid component 304 which has a concave element, is attached to the glenoid 305 via glenoid backing plate 306.

When a humeral stem 303 is used, the central region of the humeral bone must be removed to allow the humeral stem 303 to be inserted into the humerus. A cavity is thereby created in the intramedullary canal of the humerus. There are several disadvantages when using humeral stem 303 to attach prosthetic humeral head 301 to the patient's natural humerus 302. A large amount of bone needs to be removed for the insertion of the stem, weakening the bone and therefore the stem can cause the bone to fracture at implant. The bone at the centre of the patient's natural humerus is soft and therefore may cause the prosthetic humerus to sink over time.

Shoulder replacement designs of the prior art fail for a number of reasons including fracture at implant, instability, dislocation and loose humeral attachment.

Figure 3b shows an individual prosthetic humeral stem 303. This stem is shaped to fit within the intramedullary canal of the patient's humerus 302. A prosthetic humeral head 301 may be anchored on to the top of this prosthetic humeral stem 303.

### Figure 4

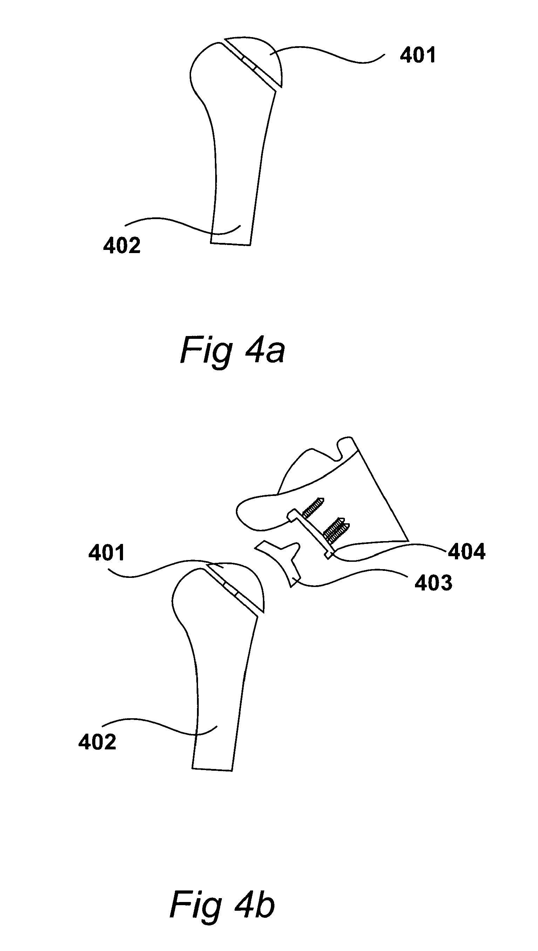

Figures 4a and 4b each show an implant assembly for a shoulder joint embodying the present invention. Figure 4a shows the joint head 401 fixed to the humerus 402.

In Figure 4a, the patient's humeral head has been resected or cut and a prosthetic humeral head 401 has been attached to the patient's humeral head by means other than a stem. As a stem has not been inserted into the humerus to allow attachment of the prosthetic humeral head to the patient's humerus, this means that the disadvantages of the prior art as described under Figure 3 are not encountered. In particular, a large amount of central humeral bone no longer needs to be removed for the insertion of the stem, thereby reducing the possibility of the a fracture of the humerus at implant. Furthermore, the joint head 401 is attached to peripheral cortical regions of the patient's humerus instead of the softer central intramedullary canal of the patient's humerus as in the prior art. The peripheral cortical regions are much harder than the central intramedullary canal of the bone and therefore there is a reduced chance of the prosthetic implant sinking over time. The implant assembly embodied by the present invention is therefore much less invasive than prior art shoulder implant assemblies.

The replacement humerus 401 shown in Figure 4a may be articulated with the patient's natural glenoid or with a prosthetic glenoid component.

Figure 4b shows a joint head 401 embodying the present invention fixed to the patient's humerus 402, articulated with a prosthetic glenoid component 403. In Figure 4b, a prosthetic glenoid component 403 and prosthetic glenoid backing plate 404 are used. The prosthetic glenoid shown in Figure 4b is a modular system. A modular system of components allows for customisation of prosthetic implants whilst enabling a minimum component set to be used by surgeons, without compromising clinical choice.

### Figure 5

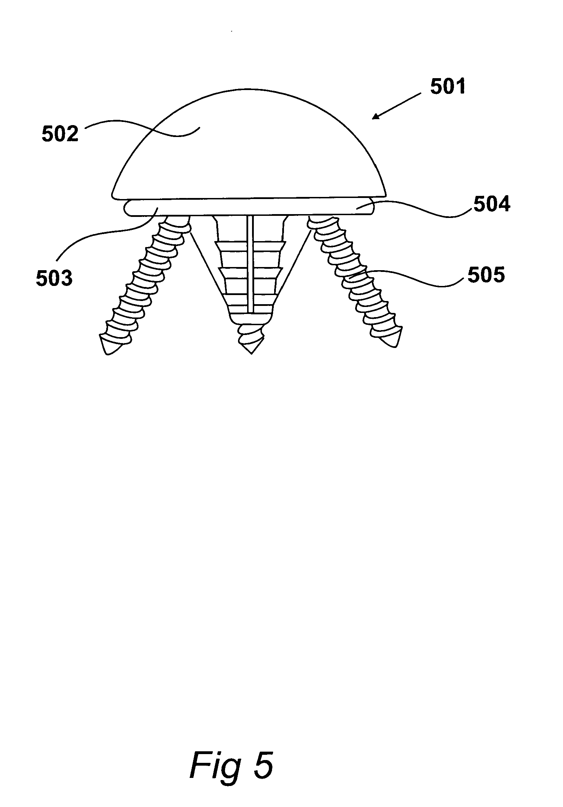

A side view of the implant assembly 501 embodying the present invention is shown in Figure 5. A joint head having a convex outer surface 502 and a generally planar base 503 is shown. Base plate 504 as shown in Figure 5 is configured with stemless means for fixation 505 to the humerus. Screws 505 are shown as the stemless means for fixation to the humerus in Figure 5. The stemless means for fixation may alternatively be pegs or anchors (not shown).

Base plate 504, in contrast to the prior art, is attached to a patient's humerus without using a humeral stem. Screws 505 are used to attach the base plate 504 to the patient's resected humeral head. The screws attach to peripheral cortical regions of the resected humeral head, which are stronger and harder than the central regions of the humeral bone. The screws 505 therefore advantageously give an improved connection to the patient's humerus over the prior art, which uses a stem to attach the prosthetic humerus to the central regions of the patient's humerus. The base plate 504 is therefore specially adapted to give good contact around the hard cortical bone of the patient's resected humerus.

### Figure 6

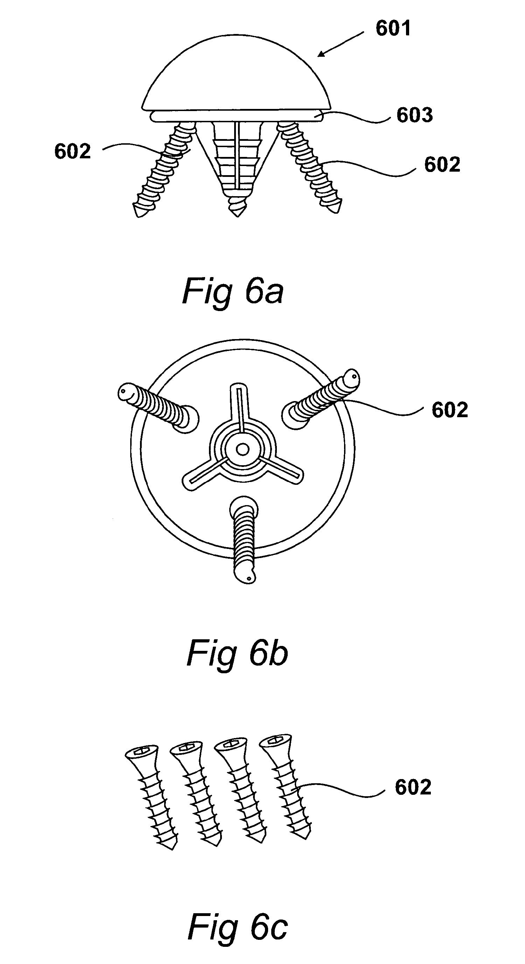

Figures 6a, 6b and 6c show screws 602 as means of attachment of the implant assembly 601 to a patient's humerus. The means of attachment of the implant assembly 601 to the patient's resected humerus may alternatively be anchors or pegs (not shown). Screws 602 shown in Figures 6a and 6b are three radially displaced screws 602 and are configured to attach the base plate 603 to the patient's humerus. Screws 602 are locked to base plate 603 with caps, providing a good fixation. In an alternative embodiment (not shown) more than three radially displaced screws may be used. Screws 602 aid the primary stability of the implant assembly 601, giving secure anchorage of the prosthetic in the patient's humerus without use of a long stem.

In contrast to the prior art in which a humeral stem is used to anchor a prosthetic humeral head to the central region of the patient's humerus, the screws 604 anchor the base plate to peripheral cortical regions of bone. The humeral bone cortex is stronger and harder than the central regions of the humeral bone, and therefore attachment to the cortex provides better support. The present invention thereby brings prosthesis stability to the periphery of the patient's humeral bone.

### Figure 7

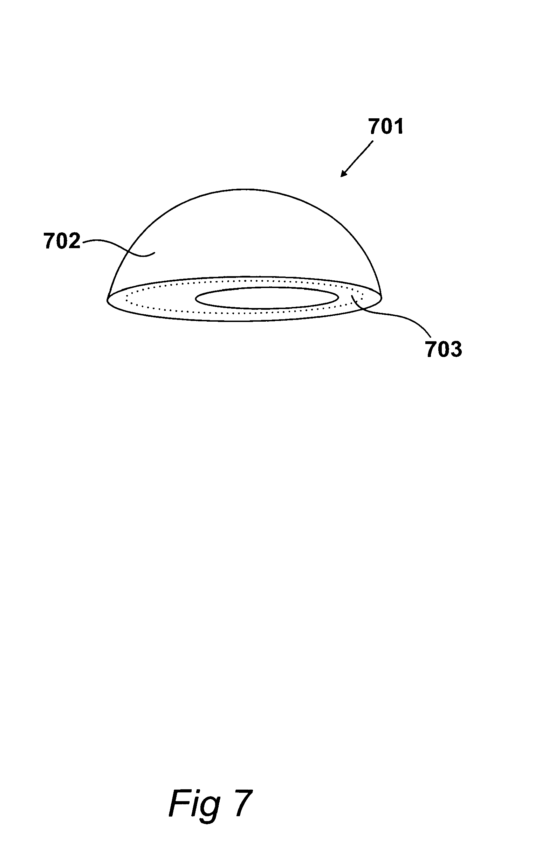

A perspective view of the joint head 701 of the implant assembly having a convex outer surface 702 and a generally planar base 703 is shown in Figure 7. The convex outer surface 702 may be hemi-spherical or hemi-ellipsoidal. The hemi-spherical or hemi-ellipsoidal nature of the joint head mimics the patient's natural humeral head. The implant assembly 701 may also be fabricated in different sizes so as to provide a range of sizes from which the surgeon can select the most appropriate size for the patient. For example, hemi-spherical stemless joint heads may be fabricated having a diameter of 42mm, 46mm, 50mm or 54mm.

The modular nature of the implant assembly embodied by the present invention therefore means that a single base plate can be used in conjunction with a number of joint heads having different sizes and shapes. This advantageously allows for a minimum number of components in the operating theatre without compromising clinical choice for the patient.

### Figure 8

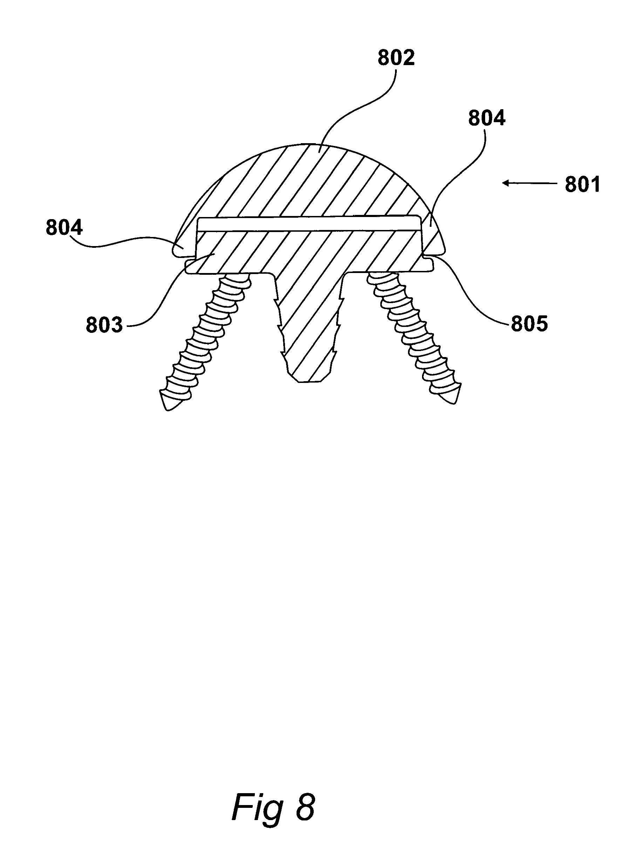

Figure 8 shows a cross sectional view of an implant assembly embodying the present invention. The modular nature of the implant assembly is apparent and as shown in Figure 8, the implant assembly 801 has two components. The implant assembly 801 comprises a joint head 802 and a base plate 803.

The joint head 802 has a convex outer surface which may be shaped so as to give rise to a hemi-spherical or hemi-ellipsoidal outer surface. The joint head 802 also has a generally planar base. The generally planar base of the joint head 802 has a lip 804 at each side. The base plate 803 has angled sidewalls.

Base plate 803 is attached to the patient's resected humeral head via stemless means. Base plate 803 is anchored to peripheral cortical regions of humeral bone. The humeral bone cortex is stronger and harder than the central regions of the humeral bone, and therefore attachment to the cortex provides better support than attachment to central regions as in the prior art. The present invention thereby brings prosthesis stability to the periphery of the patient's humeral bone.

Once base plate 803 is firmly anchored into the humeral cortex, the joint head 802 is coupled with the base plate. Each lip 804 of the generally planar base of the joint head 802 is aligned to receive the angled sidewalls of the base plate 803. The base plate 803 couples with the joint head 802 via a peripheral Morse taper 805. The joint head 802 is thus locked on to the base plate 803 by a press fit mechanism.

The diameter of the joint head 802 is greater than that of the base plate 803, and this provides an increased level of stability and security to the implant assembly. The Morse taper is internal to the joint head 802 such that the joint head 802 extends over the edge of the base plate 803 when the joint head 802 and base plate 803 are coupled together. This maximises the convex outer surface of the joint head, which advantageously increases the range of movement of the implant assembly once attached in a person's shoulder.

When base plate 803 is first attached to the patient's humerus, it must not move more than 150 micrometers. However, the means of fixation, as depicted by screws in Figure 8, provide a high level of primary stability and ensure that there is no movement of more than 150 micrometers.

The modular nature of the implant assembly 801 means that there are no screws or other means of attachment directly in the joint head 802. This advantageously means that there is no potential damage during manufacture to the glenoid-articulating surface of the joint head.

The modular nature of the implant assembly 801 also enables joint heads 802 having slightly different sizes and shapes to be used in conjunction with one base plate 803. Therefore, different joint heads 802 can be selected to suit different patients whilst using the same base plate. This advantageously allows for a minimum inventory of components without compromising clinical choice. The joint head 802 embodying the present invention can be used in association with a patient's natural glenoid or may alternatively articulate with a prosthetic glenoid. The prosthetic glenoid may also be of a modular nature.

### Figure 9

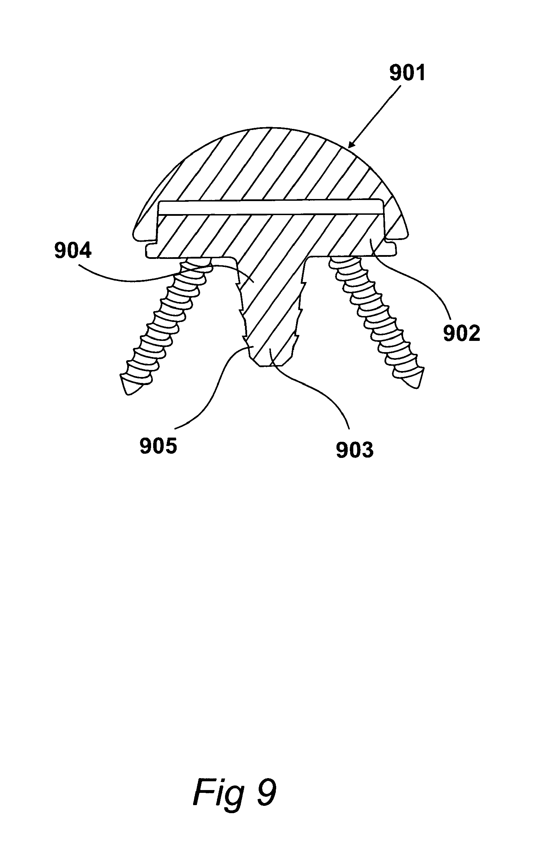

A cross sectional view of an implant assembly 901 embodying the present invention is shown in Figure 9. Base plate 902 comprises a protruding elongated portion 903 having a proximal end 904 and distal 905 end. As shown in Figure 9, the outer surface of the elongated portion is knurled to facilitate integration of the base plate 902 in the patient's resected humerus. In an alternative embodiment (not shown) the outer surface of the elongated portion may be merely roughened or include spikes or grooves. The protruding elongated portion 903 is impacted into the humeral bone.

Base plate 902 comprises titanium and is coated with hydroxy-apatite ceramic (H.A.C.). Supravit® Zoned H.A.C. may be used. Hydroxy-apatite is a calcium phosphate complex that is the primary mineral component of bone and teeth. Hydroxy-apatite ceramic is a synthetic substance that is similar to naturally occurring hydroxy-apatite found in bone and teeth. The Supravit® Zoned hydroxy-apatite ceramic coating deployed in the present invention has been clinically proven to provide high shear strength, thereby increasing the longevity of the prosthetic implant assembly. The Supravit® Zoned hydroxy-apatite ceramic coating imparts a high degree of secondary stability to the shoulder prosthetic by encouraging the development of bone growth in the resected area on the patient's humerus. It is known that the growth of bone is inhibited if any movement is present at the interface. Even very small microscopic movement will break new growth. Therefore, if growth is to be encouraged, the implant needs to be held firmly in the required position. In association with the primary stability provided by the screws or alternative means of fixation, the secondary stability provided by the hydroxy-apatite ceramic coating firmly secures the base plate embodied in the present invention in the patient's resected humerus through the encouragement of bone growth into the implant.

Joint head comprises highly polished cobalt chrome. Dome component may alternatively comprise a cobalt chrome alloy or a polymer or stainless steel.

The base plate may also comprise one or more holes (not shown), which allow for surgical repair of a fractured humerus by feeding wire through the holes to pull fragments of the fracture back around the implant. The diameter of the holes is such that they allow surgical wire to pass through, but no greater.

### Figure 10

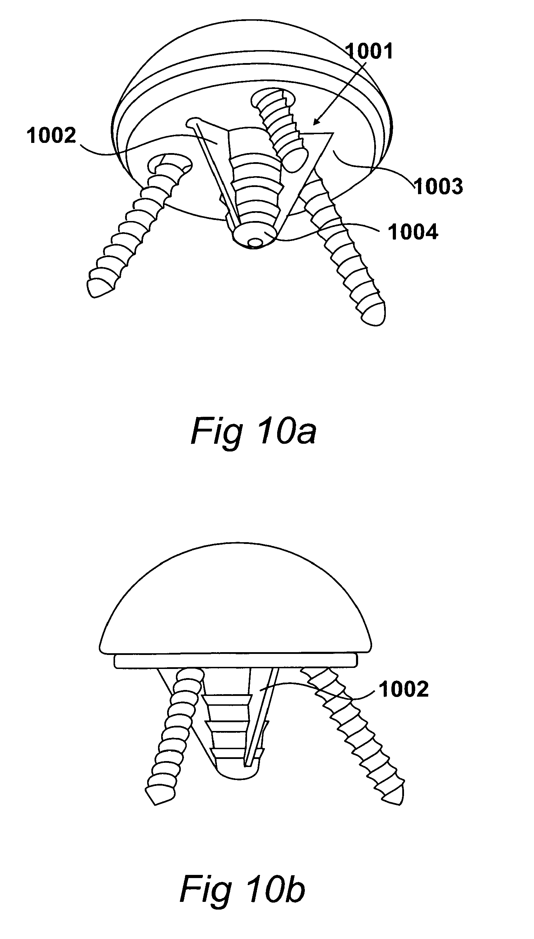

Perspective views of the implant assembly 1001 embodying the present invention are shown in Figures 10a and 10b. Three fins 1002 are positioned between the distal end 1004 of the elongated portion and the base plate 1003. These fins 1002 encourage attachment of the base plate 1003 embodied by the present invention to the resected patient's humerus. These fins 1002 are self-cutting and can cut through the humeral cortical bone to aid the secure attachment of the base plate 1003 to the patient's cortical humeral bone. Dovetail joints are used to fix the fins 1002 to the base plate 1003, providing a secure anchorage and thereby minimising the possibility of the head being pulled out of the patient's humerus.

## Claims

1. A modular implant assembly for a shoulder joint having a humerus and a glenoid, the assembly comprising;
a base plate,
a joint head having a convex outer surface and a generally planar base and which is configured to couple with said base plate,
wherein said base plate is configured with stemless means for fixation to the humerus, and further comprising at least three radially displaced screws configured to attach said base plate to the humerus, and wherein said screws attach to peripheral cortical regions of the humerus.

2. The assembly of claim 1, wherein said stemless means for fixation is a screw or a peg or an anchor.

3. The assembly of claim 1, wherein the convex outer surface of the joint head is hemi-spherical or hemi-ellipsoidal.

4. The assembly of claim 1, wherein said joint head couples with said base plate via a peripheral Morse taper.

5. The assembly of claim 4, wherein said peripheral Morse taper is internal to said joint head such that said joint head extends over the edge of said base plate.

6. The assembly of claim 1, wherein the generally planar base of said joint head has a diameter greater than the diameter of said base plate.

7. The assembly of claim 1, wherein said base plate further comprises a protruding elongated portion having a proximal end and a distal end.

8. The assembly of claim 7, wherein said elongated portion has a knurled outer surface.

9. The assembly of claim 7, wherein said elongated portion further comprises at least one fin positioned between the distal end of the elongated portion and said base plate.

10. The assembly of claim 9, comprising three radially displaced fins positioned between the distal end of the elongated portion and said base plate.

11. The assembly of claim 1, wherein the base plate comprises a material selected from the group consisting of titanium, titanium alloys and polymers.

12. The assembly of claim 1, wherein the joint head comprises a material selected from cobalt chrome, cobalt chrome alloys and stainless steels.

13. The assembly of claim 1, wherein an outer surface of said base plate comprises a coating of hydroxy-apatite ceramic.

14. The assembly of claim 1, further comprising a modular glenoid component, comprising a concave bearing member, wherein said joint head articulates with said concave bearing member.
